# EUROPEAN PATENT SPECIFICATION

(11) **EP 2 240 607 B1**
(45) Date of publication and mention of the grant of the patent: **01.04.2015**
(21) Application number: 09702463.2
(22) Date of filing: 16.01.2009
(51) Int. Cl.: C12Q 1/68

(54) **METHOD OF DETECTING AND IDENTIFYING STAPHYLOCOCCI**
VERFAHREN ZUM NACHWEISEN UND IDENTIFIZIEREN VON STAPHYLOKOKKEN
PROCÉDÉ DE DÉTECTION ET D'IDENTIFICATION DE STAPHYLOCOQUES

(30) Priority: 17.01.2008 FI 20085041; 12.02.2008 US 28044
(43) Date of publication of application: 20.10.2010
(73) Proprietor: Mobidiag Oy, 00290 Helsinki (FI)
(72) Inventor: MÄKI, Minna-Mari, 00250 Helsinki (FI); HUOPANIEMI, Laura, 02730 Espoo (FI); JÄRVINEN, Anna-Kaarina, 20500 Turku (FI); KIRVESKARI, Juha, 02300 Espoo (FI); JALAVA, Jari, 20300 Turku (FI)
(74) Representative: Kolster Oy Ab
(86) International application number: PCT/FI2009/050036
(87) International publication number: WO 2009/090310

(56) References cited:
- WO-A-2004/029299
- WO-A-2004/046379
- WO-A-2005/014857
- WO-A-2007/023461
- WO-A-2007/120808
- US-A1- 2006 286 553
- US-B1- 7 135 283
- DATABASE WPI Week 200678 Thomson Scientific, London, GB; AN 2006-760223 XP002531045 & JP 2006 271370 A (KITASATO KENKYOSHO SH) 12 October 2006 (2006-10-12) -& JP 2006 271370 A (KITASATO INST) 12 October 2006 (2006-10-12)
- DATABASE WPI Thomson Scientific, London, GB; AN 2009-B46590 XP002531047 & KR 2008 029 726 A (SAMSUNG ELECTRONICS CO LTD [KR]) 3 April 2008 (2008-04-03)

### SEQUENCE LISTING

<110> Mobidiag oy
<120> Method of detecting and identifying staphylococci
<130> 2062252PC
<160> 33
<170> PatentIn version 3.2
<210> 1
   <211> 20
   <212> DNA
   <213> Artificial
<220>
   <223> Primer
<220>
   <221> misc_feature
   <222> (3)..(3)
   <223> n represents inosine
<220>
   <221> misc_feature
   <222> (6)..(6)
   <223> n represents inosine
<220>
   <221> misc_feature
   <222> (9)..(9)
   <223> n represents G or T
<220>
   <221> misc_feature
   <222> (15)..(15)
   <223> n represents C or T
<220>
   <221> misc_feature
   <222> (18)..(18)
   <223> n represents A, C or T
<400> 1
   cgnccnggna tgtanatngg 20
<210> 2
   <211> 23
   <212> DNA
   <213> Artificial
<220>
   <223> Primer
<220>
   <221> misc_feature
   <222> (1) .. (1)
   <223> n represents A or G
<220>
   <221> misc_feature
   <222> (2)..(2)
   <223> n represents A or C
<220>
   <221> misc_feature
   <222> (3)..(3)
   <223> n represents inosine
<220>
   <221> misc_feature
   <222> (6)..(6)
   <223> n represents A or T
<220>
   <221> misc_feature
   <222> (9) .. (9)
   <223> n represents inosine
<220>
   <221> misc_feature
   <222> (12) .. (12)
   <223> n represents A or G
<220>
   <221> misc_feature
   <222> (15) .. (15)
   <223> n represents C or T
<220>
   <221> misc_feature
   <222> (18) .. (18)
   <223> n represents inosine
<220>
   <221> misc_feature
   <222> (21) .. (21)
   <223> n represents inosine
<400> 2
   nnnccnacnc cntgnagncc ncc 23
<210> 3
   <211> 20
   <212> DNA
   <213> Artificial
<220>
   <223> Primer
<400> 3
   agacctggta tgtatattgg 20
<210> 4
   <211> 20
   <212> DNA
   <213> Artificial
<220>
   <223> Primer
<400> 4
   ccaacaccat gtaaaccacc 20
<210> 5
   <211> 24
   <212> DNA
   <213> Artificial
<220>
   <223> Primer
<400> 5
   aatacaatcg cacatacatt aata 24
<210> 6
   <211> 30
   <212> DNA
   <213> Artificial
<220>
   <223> Primer
<400> 6
   ttactcatgc catacataaa tggatagacg 30
<210> 7
   <211> 21
   <212> DNA
   <213> Staphylococcus sp.
<400> 7
   tgattatggc tcaggtactg c 21
<210> 8
   <211> 21
   <212> DNA
   <213> Staphylococcus sp.
<400> 8
   tggctcaggt actgctatcc a 21
<210> 9
   <211> 21
   <212> DNA
   <213> Staphylococcus sp.
<400> 9
   tactgctatc caccctcaaa c 21
<210> 10
   <211> 21
   <212> DNA
   <213> Staphylococcus sp.
<400> 10
   attagcactt gtaagcacac c 21
<210> 11
   <211> 26
   <212> DNA
   <213> Staphylococcus sp.
<400> 11
   taacaacatg aaaaatgatt atggct 26
<210> 12
   <211> 24
   <212> DNA
   <213> Staphylococcus sp.
<400> 12
   cctcaaacag gtgaattatt agca 24
<210> 13
   <211> 25
   <212> DNA
   <213> Staphylococcus aureus
<400> 13
   aatagtatcg atgaagcatt agctg 25
<210> 14
   <211> 20
   <212> DNA
   <213> Staphylococcus aureus
<400> 14
   aacggacgtg gtatcccagt 20
<210> 15
   <211> 23
   <212> DNA
   <213> Staphylococcus aureus
<400> 15
   ggacgtggta tcccagttga tat 23
<210> 16
   <211> 23
   <212> DNA
   <213> Staphylococcus aureus
<400> 16
   tccagctgtc gaagttattt taa 23
<210> 17
   <211> 24
   <212> DNA
   <213> Staphylococcus aureus
<400> 17
   ctgtcgaagt tattttaact gttt 24
<210> 18
   <211> 23
   <212> DNA
   <213> Staphylococcus aureus
<400> 18
   gtaaaccgac agtcgaagtt atc 23
<210> 19
   <211> 20
   <212> DNA
   <213> Staphylococcus aureus
<400> 19
   cgtggtatgc caacaggtat 20
<210> 20
   <211> 24
   <212> DNA
   <213> staphylococcus aureus
<400> 20
   gtatttctat agaagataat ggac 24
<210> 21
   <211> 25
   <212> DNA
   <213> Staphylococcus epidermidis
<220>
   <221> misc_feature
   <222> (17) .. (17)
   <223> n represents A or G
<400> 21
   tagtcatatt gaagttntaa ttgag 25
<210> 22
   <211> 20
   <212> DNA
   <213> Staphylococcus epidermidis
<400> 22
   gccctgctgt cgaagttatc 20
<210> 23
   <211> 24
   <212> DNA
   <213> Staphylococcus epidermidis
<400> 23
   cattagcagg ttatgctagt cata 24
<210> 24
   <211> 20
   <212> DNA
   <213> Staphylococcus epidermidis
<400> 24
   acgccctgct gtcgaagtta 20
<210> 25
   <211> 24
   <212> DNA
   <213> Staphylococcus epidermidis
<400> 25
   tggttatggt gatgcgatta cagt 24
<210> 26
   <211> 23
   <212> DNA
   <213> Staphylococcus genus
<400> 26
   ggcattcctg ttgatattca aga 23
<210> 27
   <211> 23
   <212> DNA
   <213> Staphylococcus genus
<400> 27
   tcaacttcag aaaaaggttt aca 23
<210> 28
   <211> 23
   <212> DNA
   <213> staphylococcus genus
<400> 28
   tcaacttcag aaagaggttt gca 23
<210> 29
   <211> 23
   <212> DNA
   <213> Staphylococcus genus
<400> 29
   gttgataata gtattgacga agc 23
<210> 30
   <211> 22
   <212> DNA
   <213> Staphylococcus genus
<400> 30
   cgcccagcag ttgaagttat ct 22
<210> 31
   <211> 23
   <212> DNA
   <213> Staphylococcus genus
<400> 31
   ccagcagttg aagttatctt aac 23
<210> 32
   <211> 22
   <212> DNA
   <213> Staphylococcus genus
<400> 32
   ccagcagttg aggttatttt aa 22
<210> 33
   <211> 19
   <212> DNA
   <213> Staphylococcus genus
<400> 33
   ggggcgccca gcagttgaa 19

## Description

### FIELD OF THE INVENTION

The invention relates to a method of detecting and identifying methicillin resistant *Staphylococcus* species, and to primers and probes for use in the method. The invention further relates to a kit comprising said primers and probes.

### BACKGROUND OF THE INVENTION

Sepsis is a severe life-threatening systemic disease resulting from an infection of the bloodstream by toxin-producing bacteria. In sepsis, dysfunction of the circulatory system may lead to multiple organ failures and to a drop in the blood pressure, resulting in shock. Worldwide, sepsis is the most common cause of death in intensive care units with a mortality rate ranging from 20% to 60%.

Staphylococcus species are one of the sepsis causing pathogens. Sepsis cases in which the causative agent is either *Staphylococcus aureus* or methicillin-resistant *Staphylococcus aureus* (MRSA) are associated with a mortality rate of 15% to 30%. Clinical data suggest that 28% of patients with S. *aureus* sepsis do not receive early and appropriate treatment and that 10% of patients may initially be receiving inappropriate antibiotic treatment.

In the treatment of sepsis, early identification of causative agents and the antibiotic resistance markers is essential in the re-assessment of empirical antimicrobial therapy. The empirical therapy chosen is often vancomycin due to the increased methicillin resistance of staphylococci. The efficacy of vancomycin therapy can, however, be suboptimal for methicillin-sensitive *S.aureus* (MSSA) due to pharmacokinetic and pharmacodynamic reasons. High mortality rates associated with sepsis caused by *S*. *aureus* and MRSA emphasize the importance of identification of the methicillin resistance marker gene *mecA.*

Bacteria of staphylococcus genus are Gram-positive cocci-form bacteria. Most of the staphylococcus species are coagulase-negative bacteria, with the exception of *S*. *aureus, S. intermedius, S. delphini,* and S. *schleiferi* sub-species *coagulans.* The coagulase-negative staphylococci (CoNS) remain an important cause of sepsis in hospitalized patients. Among these CoNS, the methicillin resistance is prevalent (Marshall et al. Diagn. Microbiol. Infect. Dis. 1998,

Methicillin resistance in *Staphylococcus* species arises by acquisition of a large genetic element called the staphylococcal cassette chromosome SCCmec. The SCCmec cassette carries the *mecA* gene, which encodes penicillin binding protein PBP 2a that determines methicillin resistance. Five different types of SCCmec cassettes including their variants have been identified. However, in these cassettes the *mecA* gene is highly conserved among *Staphylococcus* species.

Methods for detecting methicillin resistance are based on either the phenotypic or genotypic characterization. Many strains of staphylococci, however, express the *mecA* gene heterogeneously. Thus, the phenotypic detection of methicillin resistance is problematic and unreliable, especially in CoNS (Hussain et al., J. Clin. Microbiol. 2002, 40:2251-2253). Therefore, genotypic characterization, based on identification of *mecA* gene in the genome of staphylococci, is more accurate when determining the methicillin resistance. Furthermore, genotypic characterization is faster than phenotypic methods, which require the cultivation period of staphylococci. For example, European patent publication EP0887424B1 discloses a method for detecting the presence of an MRSA or MRCoNS (methicillin-resistant coagulase-negative staphylococci). In the method, a reaction is performed with a sample by making a combined use, as primers and/or a probe, of a part of a *mecA* gene integrated in staphylococcal genome, and a part of a base sequence of a chromosomal DNA surrounding said integrated DNA.

International patent publication WO 2007/023461 discloses primers and probes useful in detecting micro-organisms based on 23S rRNA gene and antibiotic resistance genes including *mecA.* The method relies on specific primer pairs to amplify 23S rRNA from different bacterial species. Thus, a panel on pathogens can be simultaneously identified using multiplex PCR, which can be demanding to optimize for all primer pairs. The use of ribosomal RNA also includes some drawbacks. Distinguishing related bacterial species from each other with the help of rRNA molecules can be difficult, because the sequences of these molecules do not contain enough variable regions when they are compared with each other.

International patent publication WO 2004/046379 discloses primers and probes useful in a diagnostic method of detecting and identifying bacterial species which cause infections, particularly respiratory tract infections. Said detecting and identifying is based on broad-range primers that originate from conserved regions of genes encoding topoisomerases, especially the gyrB/parE genes, and bacterial species-specific oligonucleotide probes that originate from hyper-variable regions situated between the conserved regions. Some of the probes are specific for *Staphylococcus aureus.*

Thus, there remains a need in the art for sensitive methods of detecting methicillin resistant *Staphylococcus* species.

### BRIEF DESCRIPTION OF THE INVENTION

The present invention provides a method of detecting and identifying methicillin resistant *Staphylococcus* species in a sample, as defined in claims 1-8.

In some embodiments, the method may comprise contacting the amplified DNA with all or any combination of the S. aureus-species-specific probes depicted in SEQ ID NOs: 13 to 20, and/or with all or any combination of the mecA-specific probes depicted in SEQ ID NOs: 7 to 12.

In one embodiment, the method further comprises contacting the amplified DNA with at least one *Staphylococcus epidermidis-species-*specific oligonucleotide probe hybridizing with the amplified topoisomerase genes. In one particular embodiment, said probe comprises a sequence selected from a group consisting of SEQ ID NOs: 21 to 25 and complementary or reversed sequences thereof. The method may also comprise contacting the amplified DNA with all *S*. *epidermidis-specific* probes depicted in SEQ ID NOs: 21 to 25.

in another embodiment, the first primer pair comprises at least one sequence depicted in SEQ ID NOs: 1 or 2 or in complementary or reversed sequences thereof. In still another embodiment, the second primer pair comprises at least one sequence selected from SEQ ID NOs: 5 and 6 and complementary or reversed sequences thereof.

In a further embodiment, said at least one staphylococcus genus-specific probe comprises a sequence selected from a group consisting of SEQ ID NOs: 26 to 33 and complementary or reversed sequences thereof. The method may also comprise contacting the amplified DNA with all genus-specific probes depicted in SEQ ID NOs: 26 to 33.

The method according to the present invention may further comprise DNA amplification with at least one *S. aureus*-species-specific primer hybridizing with conserved regions of genes encoding topoisomerases for example to enhance amplification of *S*. *aureus* DNA. In one embodiment, said primer comprises a sequence selected from SEQ ID NOs: 3 and 4 and complementary or reversed sequences thereof. In another embodiment both primers depicted in SEQ ID NOs: 3 and 4 are used in the method.

Disclosed is that, where the sample to be analyzed is *S*. *aureus* pure culture, said first primer pair comprises at least one sequence selected from SEQ ID NOs: 3 and 4 and reversed or complementary sequences thereof. Both primers depicted in SEQ ID NOs: 3 and 4 may be used in the method. DNA amplification with broad-range primers hybridizing with conserved regions of genes encoding topoisomerases may be omitted. When desired, contacting the amplified DNA with the staphylococcus genus-specific oligonucleotide probes may also be omitted

The method according to the present invention may be performed in a solution or on a solid support. Thus, in one embodiment, said primers and/or probes are attached onto a solid support, such as a biochip. The biochip may comprise separate compartments for the primers and the probes.

Furthermore, the method may consist of the steps described above or it may comprise for example DNA amplification with additional primers and/or contacting the amplified DNA with additional probes. In some embodiments, the method may also comprise further steps, such as washing and incubation steps.

The present invention also provides mecA-specific oligonucleotide probes, as defined in claim 9, consisting of a nucleotide sequence selected from a group consisting of SEQ ID NOs: 7 and 9 to 12 and complementary sequences thereof.

In addition, the present invention provides *S*. *aureus-*species-specific oligonucleotide probes, as defined in claim 10, consisting of a sequence selected from a group consisting of SEQ ID NOs: 13 to 16 and 19-20 and complementary sequences thereof.

The present invention further provides a kit for use in detecting and identifying methicillin resistant *Staphylococcus* species in a sample, as defined in claim 11. The kit comprises at least one toposiomerase-specific primer, at least one *mecA*-specific primer, at least one *S. aureus*-species-specific topoisomerase probe comprising a sequence selected from a group consisting of SEQ ID NOs: 13 to 20 and complementary sequences thereof, and at least one *mecA*-specific oligonucleotide probe comprising a sequence selected from a group consisting of SEQ ID NOs: 7 to 12 and complementary sequences thereof; and at least one staphylococcal genus-specific topoisomerase probe comprising a sequence selected from a group consisting of SEQ ID NOs:26 to 33 and complementary sequences thereof. In some embodiments, the kit may comprise all or any combination of the *S. aureus*-species-specific toposiomerase probes depicted in SEQ ID NOs: 13 to 20, and/or all or any combination of the *mecA*-specific oligonucleotide probes depicted in SEQ ID NOs: 7 to 12.

One embodiment comprises at least one toposiomerase-specific primer comprising a sequence depicted in SEQ ID NO: 1 or SEQ ID NO: 2 or in complementary or reversed sequences thereof. Optionally, the kit may further comprise at least one *S. epidermidis*-species-specific topoisomerase probe. Said *S. epidermidis*-species-specific probe may comprise a sequence selected from a group consisting of SEQ ID NOs: 21 to 25 and complementary or reversed sequences thereof.

The kit according to the present invention may contain *S*. *aureus*-species-specific primer additional to broad-range toposiomerase primers in the kit. Said *S. aureus*-species-specific primers may comprise a sequence selected from SEQ ID NOs: 3 and 4 complementary or reversed sequences thereof.

In another embodiment, the kit comprises at least one *mecA-*specific primer comprising a sequence selected from SEQ ID NOs: 5 and 6 and complementary or reversed sequences thereof. The kit may also comprise both primers depicted in SEQ ID NOs: 5 and 6.

The kit according to the present invention may further comprise a biochip in which said primers and probes are contained and/or to which the same are attached. The biochip may comprise separate compartments for the primers and the probes.

Furthermore, the kit according to the present invention may consist of the primers and probes described above and, optionally, of the biochip, or it may comprise additional primers and/or probes, as well as appropriate reagents for performing for example DNA amplification and hybridization.

### BRIEF DESCRIPTION OF THE DRAWINGS

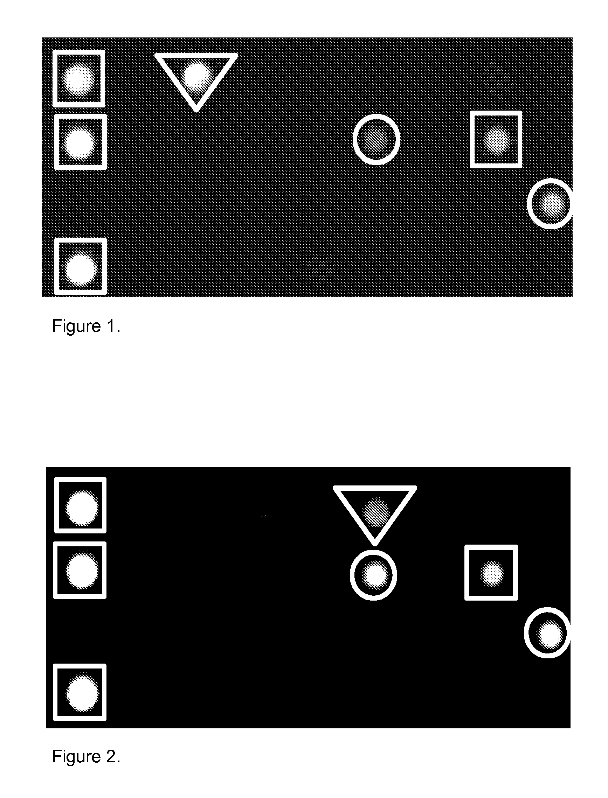

In the following, the invention will be described in greater detail by means of preferred embodiments with reference to the attached drawings, in which
Figure 1 illustrates simultaneous detection of *S*. *aureus* (triangle-marked spot; SEQ ID NO: 18) and a *mecA* gene (circled spots; SEQ ID NOs: 7 and 9, SEQ ID NOs: 8 and 10) on a microarray. The *mecA* spots on the microarray comprised of two specific *mecA* oligos per spot. Four control oligonucleotides were also detected (squared spots).
Figure 2 illustrates simultaneous detection of bacteria of staphylococcus genus (triangle-marked spot; SEQ ID NO: 26) and a *mecA* gene (circled spots; SEQ ID NOs: 7 and 9, SEQ ID NOs: 8 and 10) on a microarray. The *mecA* spots on the microarray comprised of two specific *mecA* oligos per spot. Four control oligonucleotides were also detected (squared spots).

### DETAILED DESCRIPTION OF THE INVENTION

The present invention provides a method for molecular identification of methicillin resistant *Staphylococcus* species using gene sequences from topoisomerase and methicillin resistance marker genes.

The method is based on identifying the presence of staphylococcal DNA other than *S*. *aureus* in a sample by using genus-specific probes, and the presence of *S*. *aureus* DNA in the same sample by using species-specific probes. In addition, the presence of a methicillin resistance (*mecA*) gene in the sample is detected using mecA-specific probes. By analyzing the formed hybridization complexes, if any, the method provides a detailed analysis of the sample in a single assay. For instance, the method can be used to determine infection or contamination of the sample with *mecA* negative staphylococci other than *S*. *aureus, mecA* positive staphylococci other than *S*. *aureus, mecA* positive *S.aureus* and/or *mecA* negative *S*. *aureus.*

The use of *mecA*-specific probes in combination with bacterial genus- or species-specific probes designed to hybridize with a topoisomerase gene, especially *gyrB* or *parE,* which is considerably more variable than for example a 16S/23S rRNA gene region previously used in bacterial diagnostics, allows simultaneous detection of methicillin resistance and infection-causing bacteria. Thus, the method of the present invention does not suffer from the problems of the prior art. An amplification step incorporated in the method provides high sensitivity. The *gyrB* and *parE* genes from phylogenetically different bacterial species are amplified efficiently with broad-range primers regardless of the bacterial species being gram-negative or gram-positive. The simultaneous amplification of *mec.4* provides information on methicillin resistance of the identified bacteria.

Topoisomerase genes gyrase B (*gyrB*) and *parE* contain species-specific hyper-variable regions which can be used for identifying different bacterial species. The *GyrB* gene exists as a single copy in all bacteria, while the *parE* gene exists mainly in gram-negative bacteria, also as a single copy. In the method of the present invention, a topoisomerase genes, especially *gyrB* and *parE*, are used to identify *Staphylococcus* species. This is achieved by amplifying the bacterial hyper-variable topoisomerase genes in a sample by using primers comprising a sequence which hybridizes with conserved gene regions flanking said hyper-variable region, and identifying said amplified gene region with genus or species-specific probes. The *gyrB* and *parE* genes are amplifiable by the same primers.

Amplification of the staphylococcal hyper-variable topoisomerase genes, especially *gyrB* and *parE,* may be performed using at least one broad-range primer, or broad-range primer mixture, comprising a sequence depicted in SEQ ID NOs: 1 or 2. The amplification may also be performed using a primer comprising at least one sequence depicted in SEQ ID NO: 1 and a second primer comprising at least one sequence depicted in SEQ ID NO: 2. In some embodiments, at least one *S. aureus*-specific primer is used to enhance the amplification of the hyper-variable topoisomerase genes of *S*. *aureus.* In one particular embodiment, said *S. aureus*-specific primer is selected from a group consisting of SEQ ID NOs: 3 and 4. In another embodiment, both *S*. *aureus*-specific primers are used in the amplification.

The method according to the present invention comprises detection and identification of staphylococci with at least one genus-specific probe (SEQ ID NOs: 26 to 33) designed to hybridize with the hyper-variable region of a topoisomerase gene, especially *gyrB* or *parE,* of staphylococcus species. In one embodiment of the present invention, at least one probe comprising a sequence selected from a group consisting of SEQ ID NOs: 26 to 33 is used for identifying staphylococcus species. These genus-level probes were designed to identify all staphylococcus species other than *S*. *aureus.* In other embodiments, more than one of said genus-specific probes is used for said identification.

Effective antimicrobial therapy requires a distinction between *S*. *aureus* and other staphylococcus species. Accordingly, the method comprises detection and identification of *S*. *aureus* by a species-specific probe designed to hybridize with the hyper-variable region of a topoisomerase gene, especially *gyrB* or *parE,* of *S*. *aureus.* In one embodiment, at least one probe comprising a sequence selected from a group consisting of SEQ ID NOs: 13 to 20 is used for detecting and identifying *S*. *aureus.*

Since *S*. *epidermidis* is the most common CoNS isolated in clinical laboratories, optionally the method may also comprise species-specific identification of *S*. *epidermidis.* This may be achieved by using specific probes designed to hybridize with the hyper-variable region of a topoisomerase gene, especially *gyrB* or *parE',* of *S*. *epidermidis.* In one embodiment, at least one probe comprising a sequence selected from a group consisting of SEQ ID NOs: 21 to 25 is used for detecting and identifying *S*. *epidermidis.*

The method according to the present invention further comprises determining the presence of methicillin resistance in a sample by employing a *mecA* gene. First, a *mecA* gene is amplified using appropriate primers. In one embodiment, at least one primer comprising a sequence selected from SEQ ID NOs: 5 and 6 is used for the amplification. In other embodiments, a first primer comprising a sequence depicted in SEQ ID NO: 5 and a second primer comprising a sequence depicted in SEQ ID NO: 6 are used in the amplification. The possibly amplified *mecA* gene is then detected using *mecA* specific oligonucleotide probes. In one embodiment, said detection is performed with at least one probe comprising a sequence selected from a group consisting of SEQ ID NOs: 7 to 12.

The term "sample" as used herein encompasses a variety of sample types including bacterial and cell cultures, food samples, soil samples, biological fluids, and clinical specimens, such as a tissue sample, a secretion sample, such as sputum, or a brush sample, a blood sample, or another suitable sample, obtained from a patient suspected of suffering from an infectious disease. Especially, the sample to be analyzed is a positive blood culture sample suitable for clinical diagnostic applications.

Disclosed is that, wherein the sample to be analyzed is a pure culture of *S*. *aureus,* a hyper-variable region of topoisomerase genes, especially *gyrB* and *parE,* is amplified using *S. aureus*-species-specific primers, such as those depicted in SEQ ID NOs: 3 and 4, hybridizing with conserved regions of said topoisomerase.

A person skilled in the art understands that the method may be easily expanded by contacting the amplified DNA with additional species- or genus-specific probes hybridizing with the amplified topoisomerase genes. The method may also comprise additional steps, such as washing or incubation.

In the method according to the present invention, the toposiomerase and *mecA* genes may be amplified simultaneously for example by a PCR reaction. Prior to amplification, DNA may be isolated from a sample to be analyzed by any conventional method, such as commercial DNA isolation kits (e.g., Roche High Pure PCR Template Preparation Kit, BD Biosciences Clontech NucIeoSpin®, or Qiagen QIAamp® DNA Mini kit) or by traditional extraction with phenol-chloroform or equivalent organic solvents, either manually or with special devices that are suitable for performing DNA isolation. Automated sample preparation platforms for the isolation of DNA (e.g., bioMérieux NucliSENS® easyMAG™) are preferably used because of their general availability, rapidity, and repeatability. The DNA can also be obtained directly from the sample to be analyzed without separate isolation.

In order to form a single-stranded target strand, any suitable method can be used including asymmetric PCR, or exonuclease treatment. The invention also comprises applications in which a double-stranded PCR product can be used in the hybridization reaction e.g. after a denaturation step.

A number of known labeling methods can be used in the method according to the present invention in order to produce a labeled target strand, for instance fluorescent labels (e.g., Cy5, Cy3, Cy2, TexasRed, FITC, Alexa 488, TMR, FluorX, ROX, TET, or HEX), radioactive labels (e.g., 32P, 33P, or 33S), chemiluminescent labels (e.g., HiLight Single-Color Kit), and colorimetric detection (e.g., biotin-streptavidin-enzyme conjugate). The invention also encompasses applications in which no label is needed, such as those in which the detection of nucleic acids is based on electric impulses (e.g., Motorola eSensor™).

In the method of the present invention, reagents used in the DNA amplification can be any reagents that are conventionally used for the amplification of DNA, and are well known to the persons skilled in the art. Suitable and cost-effective reagents, which are commercially available, include different types of DNA polymerases and buffers therefore (e.g., AmpliTaqGOLD™, AmpliTaq® LD, DyNAzyme™, TaqPlus® Precision, or HotStart-Taq®) nucleotides or pre-prepared mixtures of nucleotides (e.g., Sigma, Applied Biosystems, or Amersham Biosystems), and MgCl₂ (whereby a product from the manufacturer of the DNA polymerase is generally used).

The equipment used for amplification can be any suitable device (e.g., Biometra® T1 Thermocycler, Applied Biosystems GenAmp® PCR system 2700, or Eppendorf Mastercycler®). Practically all devices and equipment suitable for DNA amplification can be used, and amplification can also be performed manually by transferring reaction tubes from one temperature to another. In addition, amplification can be performed directly on a biochip containing specific wells for the PCR reaction, and specific hybridization area, such as a microarray, whereto the probes and control oligonucleotides can be attached.

When necessary, purification of the PCR product can be performed by any commercial method (e.g., Roche High Pure PCR Product Purification Kit, Amersham Biosciences MicroSpin™ S-400 or S-300 HR Columns, or Qiagen QIAquick® PCR-Purification-Kit) or using extraction with an organic solvent. The amplification product can also be used for the hybridization reaction as such without any purification or extraction steps.

In the hybridization reaction, each single-stranded probe hybridizes with its single-stranded target strand creating a double-stranded structure. In some embodiments the probe and the target strand hybridizes under normal hybridization conditions. A person skilled in the art is able to determine suitable hybridization conditions. The hybridization may be carried out at various hybridization temperatures (generally between 40°C and 70°C), and the time needed to perform the hybridization may vary, depending on the application, from a few minutes to a few days.

When hybridization takes place on a solid support, the probes used in hybridization can be attached onto the surface of the solid support by covalent or non-covalent binding. Alternatively, other chemical, electrochemical or equivalent attachment methods can be used. The substrate or support, to which the probes are attached can be manufactured from glass, plastic, polymers, metal, nylon, nitrocellulose, polyacrylamide, silicon, silicon oxide, silica, or a combination thereof, and the size of the substrate can vary from a couple of millimeters to a few centimeters. The surface of the substrate can be coated with aminosilane or other appropriate surface treatment, such as epoxysilane, or alternatively a substrate that does not require any separate surface treatment can be used. Preferred substrates for the oligonucleotide probes are glass and silicon.

The synthesized probes can be printed onto the surface of the microarray support with any commercially available arrayer that is suitable for this purpose (e.g., Qarray-mini arraying system, Lucidea Array Spotter, OmniGrid® arrayer) or they can be pipetted manually onto the surface. Alternatively, the probes can be synthesized directly onto the surface by an appropriate *in situ* synthesis method, such as photolithography or ink-jet technology.

The mixture used in hybridization may have a composition different from that presented below in the working Examples, for example, the salt composition and/or the concentration may vary (e.g., 2-4xSSC, or SSPE), or commercially available hybridization solutions can be used (e.g., Sigma ArrayHyb™). In addition, denaturing or stabilizing additives (e.g., formamide, or dimethyl sulfoxide (DMSO)) or substances that decrease non-specific binding (e.g., bovine serum albumin (BSA), or salmon sperm DNA) can be used in the hybridization mixture. Hybridization can be carried out at various hybridization temperatures (generally between 40°C and 70°C), and the time needed to perform hybridization can vary, depending on the application, from a few minutes to a few days. Instead of a water bath, hybridization can be carried out for example in an incubator or in a special hybridization device (e.g., GeneTAC HybStation, or Lucidea Slidepro Hybridizer). The post-hybridization washing steps can vary in their duration, volume, temperature, and in the composition of the washing solution, and can therefore differ from the exemplified method. The washing of the microarray can also be performed in a separate device. In some cases, washing is not necessary because the microarray can be analyzed immediately after hybridization. In a preferred method, hybridization is performed in a special hybridization device or incubator at 53°C to 58°C for about 15 to 30 minutes.

Hybridization complexes formed between the amplified DNA and the probes according to the present invention, if present, can be detected using any suitable method by which hybridization under normal conditions can be demonstrated, for instance Southern blot, Northern blot, colony hybridization, fluorescence *in situ* hybridization (FISH), sequencing, and RT-PCR methods. These methods are well known to the persons skilled in the art and they can be performed both in a solution and on a solid support that binds DNA, such as on a nitrocellulose or nylon membrane or on a glass or silicon based surface.

The microarray can be analyzed by any equipment or reader applicable to this purpose (e.g., GeneTAC UC4, GenePix® Personal 4100A, Agilent DNA Microarray Scanner, or a reader which consists of a light source and a camera). If the target strand is fluorescently labeled, the analysis can also be performed for example by a fluorescent microscope. If a radioactive label has been used, the array or membrane can be analyzed by autoradiography. If hybridization has been carried out on the surface of an electronic microarray and the analysis is thus based on electronic detection, the microarray can be analyzed by special equipment designed for this purpose.

The broad-range primers for use in the present invention (Table 1; SEQ ID NOs: 1 and 2) were designed using sequences from the EMBL public sequence database, or, in cases where such sequences were not available in public databases, they were produced by cloning the topoisomerase gene sequence fragments from the bacterial species, or they were produced by PCR with primers designed using sequences from closely related bacterial species or partial sequences available in the databases.

**Table 1. Broad-range primers used in amplification reaction**

| Primer | Gene | SEQ ID NO: | Sequence 5'->3' |
|---|---|---|---|
| gB3F | gyr*B*/*parE* | 1 | CGICCIGGKATGTAYATHGG |
| gB4R | *gyrB*/*parE* | 2 | RMICCWACICCRTGYAGICCICC |

In the primer sequences, I represents base inosine; K represents base G or T; Y represents base C or T; H represents base A or C or T; R represents base A or G; M represents base A or C, and W represents base A or T. The broad-range primer mixtures for use in the present invention consist of several different primer alternatives. For example, in the case of primer SEQ ID NO: 1, the mixture includes primers in which K represents G (guanine) or T (thymine).

The most successful amplification of pure bacterial DNA was achieved with one of a plurality of different primer pair mixtures tested by various laboratory tests. The *gyrB* and *parE* genes of all tested bacteria could be amplified with the primer mixture (Table 4). The primers thus act as universal or broad-range primers for the bacteria. The bacterial species from culture collections (ATCC and DSM) along with clinical samples were used in the specificity and sensitivity testing.

The present invention further provides S. *aureus-specific* primers which hybridize with conserved regions of genes encoding topoisomerases, especially *gyrB* and *parE,* (Table 2; SEQ ID NOs: 3 and 4) of S. *aureus.* These primers were designed as described above in connection with the broad-range primers.

**Table 2. S. aureus-specific primers**

| Primer | Gene | SEQ ID NO: | Sequence 5'->3' |
|---|---|---|---|
| SaureusF | *gyrB*/*parE* | 3 | AGACCTGGTATGTATATTGG |
| SaureusR | *gyrB*/*parE* | 4 | CCAACACCATGTAAACCACC |

Furthermore, the present invention provides primers capable of amplifying a *mecA* gene (Table 3; SEQ ID NOs: 5 and 6). These primers were designed as described above in connection with the broad-range primers but instead of using *gyrB* or *parE* gene sequences, the *mecA* gene from multiple staplylococcus species was applied.

**Table 3. mecA-specific primers**

| Primer | Gene | SEQ ID NO | Sequence 5'->3' |
|---|---|---|---|
| mecAF | *mecA* | 5 | AATACAATCGCACATACATTAATA |
| mecAR | *mecA* | 6 | TTACTCATGCCATACATAAATGGATAGACG |

In some embodiments, the present invention provides a mixture of primers comprising any combination of broad-range primers, *S*. *aureus-*specific primers, and *mecA* primers comprising a sequence selected from a group consisting of SEQ ID NOs: 1 to 6, respectively.

The broad-range primers, *S. aureus*-specific primers, as well as *mecA* primers described above include oligonucleotide analogues, such as peptide nucleic acids (PNA), and oligonucleotides comprising modified bases. The primers may consist of nucleotide sequences depicted in respective SEQ ID NOs or they may comprise additional nucleotides. Furthermore, the primers do not need to be of exactly the same sequence as the template nucleic acid but must be sufficiently complementary to hybridize with the template and retain the capability to amplify the topoisomerase or mecA region. Also, various chemical compounds or groups (e.g., amino groups) or other molecules, such as labels, can be attached to the primers, or they can be entirely unmodified. Naturally, reverse and complementary sequences of these oligonucleotide sequences are equally suitable, as is obvious to a person skilled in the art.

Herein the term "complementary sequence" refers to a nucleic acid sequence which consists of complementary nucleotides to the original primer sequence. As known in the art, nucleic acids A and T are complementary to each other, whereas nucleic acids C and G are complementary to each other. In other words, the complementary sequence to T-T-C-A-G (where each letter stands for one of the bases in DNA) is A-A-G-T-C. Thus, the term "complementary sequence" refers to a completely complementary sequence which consists of as many nucleic acids as the original primer sequence.

The term "reverse sequence" refers to a nucleic acid sequence in which the order of nucleotides is reversed as compared to the original primer sequence. For example, reverse sequence to T-T-C-A-G (where each letter stands for one of the bases in DNA) is G-A-C-T-T. Thus, the term "reverse sequence" refers to a completely reversed nucleic acid sequence consisting of as many nucleic acids as the original non-reversed primer sequence.

Thus, each specific SEQ ID NO also refers to reversed and complementary sequences thereof. Similarly, functional fragments of the previously mentioned oligonucleotide sequences are useful as primers. In this context, the term "functional fragment" refers to a truncated nucleotide sequence with or without modifications, capable of hybridizing with and initiating amplification of the target DNA.

Disclosed is a mixture of primers comprising any combination of the primers according to the present invention.

In the design of staphylococcal genus-specific, S. *aureus-*species-specific, as well as *S. epidermidis*-species-specifiic oligonucleotide probes, a planning strategy based on alignment was used. The *gyrB* or *parE* genes of target bacterial species were aligned with the corresponding genes of the non-targeted bacteria. The sequences were obtained from the EMBL public sequence database or, in cases where such sequences were not available in public databases, they were produced by cloning the *gyrB* or *parE* gene sequence fragments from the bacterial species, or they were produced by PCR with primers designed using sequences from closely related bacterial species or partial sequences available in the databases. The specificity of the oligonucleotide probes was tested in laboratory conditions both with pure DNA samples isolated from various bacterial species and with clinical patient samples (Table 4).

**Table 4. Staphylococcus species used in designing and testing genus- and species-specific probes and primers**

| Staphylococcus species | Supplier's code (type of sample) |
|---|---|
| *S*. *aureus* | ATCC 29247, ATCC 51153, ATCC 49333, ATCC 43300 |
| *S*. *capitis* | ATTC 27840, ATCC 27842 |
| *S*. *cohnii* | ATCC 29972, ATCC 29974 |
| *S. epidermidis* | ATCC155-U |
| *S. haemolyticus* | DSM 20263 |
| *S*. *hominis* | ATCC 25615, ATCC27844 |
| *S*. *intermedius* | ATCC 29663 |
| *S*. *lugdunensis* | DSM 4805 |
| *S*. *saccharolyticus* | ATCC14953 |
| *S*. *sciuri* | ATCC 29060, ATCC 29062, ATCC 29059 |
| *S*. *warneri* | ATCC 17917, ATCC 27836, ATCC 27837, ATCC 25614 |
| *S*. *xy*/*osus* | ATCC35033 |

The present invention further provides *mecA*-specific oli-gonucleotide probes (Table 5; SEQ ID NOs: 7 and 9 to 12) designed based on alignment of staphylococcal strains containing *mecA* gene. These probes were designed as described above in connection with topoisomerase probes.

**Table 5. mecA probes**

| SEQ ID NO: | Sequence (5'-3') |
|---|---|
| 7 | TGATTATGGCTCAGGTACTGC |
| 8 | TGGCTCAGGTACTGCTATCCA |
| 9 | TACTGCTATCCACCCTCAAAC |
| 10 | ATTAGCACTTGTAAGCACACC |
| 11 | TAACAACATGAAAAATGATTATGGCT |
| 12 | CCTCAAACAGGTGAATTATTAGCA |

Suitable probes for use in the present invention hybridizing with the amplified topoisomerase genes include those disclosed in Table 6. Probes comprising a sequence selected from the group consisting of SEQ ID NOs: 13 to 20 and complementary or reversed sequences thereof are specific for *S*. *aureus.* Probes comprising a sequence selected from a group consisting of SEQ ID NOs: 26 to 33 and complementary or reversed sequences thereof are specific for staphylococcus genus. Probes comprising a sequence selected from a group consisting of SEQ ID NOs: 21 to 25 and complementary or reversed sequences thereof are specific for *S*. *epidermidis.* These probes and combinations thereof are used in different embodiments of the present invention.

**Table 6. topoisomerase probes**

| SEQ ID NO: | Gene | Specificity | Sequence (5'-3') |
|---|---|---|---|
| 13 | *gyrB* | S. aureus | AATAGTATCGATGAAGCATTAGCTG |
| 14 | *gyrB* | S. aureus | AACGGACGTGGTATCCCAGT |
| 15 | *gyrB* | S. aureus | GGACGTGGTATCCCAGTTGATAT |
| 16 | *gyrB* | S. aureus | TCCAGCTGTCGAAGTTATTTTAA |
| 17 | *gyrB* | S. aureus | CTGTCGAAGTTATTTTAACTGTTT |
| 18 | *parE* | S. aureus | GTAAACCGACAGTCGAAGTTATC |
| 19 | *parE* | S. aureus | CGTGGTATGCCAACAGGTAT |
| 20 | *parE* | S. aureus | GTATTTCTATAGAAGATAATGGAC |
| 21 | *gyrB* | S. epidermidis | TAGTCATATTGAAGTTRTAATTGAG |
| 22 | *gyrB* | S. epidermidis | GCCCTGCTGTCGAAGTTATC |
| 23 | *gyrB* | S. epidermidis | CATTAGCAGGTTATGCTAGTCATA |
| 24 | *gyrB* | S. epidermidis | ACGCCCTGCTGTCGAAGTTA |
| 25 | *parE* | S. epidermidis | TGGTTATGGTGATGCGATTACAGT |
| 26 | *gyrB* | S. genus | GGCATTCCTGTTGATATTCAAGA |
| 27 | *gyrB* | S. genus | TCAACTTCAGAAAAAGGTTTACA |
| 28 | *gyrB* | S. genus | TCAACTTCAGAAAGAGGTTTGCA |
| 29 | *gyrB* | S. genus | GTTGATAATAGTATTGACGAAGC |
| 30 | *gyrB* | S. genus | CGCCCAGCAGTTGAAGTTATCT |
| 31 | *gyrB* | S. genus | CCAGCAGTTGAAGTTATCTTAAC |
| 32 | *gyrB* | S. genus | CCAGCAGTTGAGGTTATTTTAA |
| 33 | *gyrB* | S. genus | GGGGCGCCCAGCAGTTGAA |

| | | | |
|---|---|---|---|
| In the SEQ ID NO: 21, R represents base A or G. | | | |

The oligonucleotide probes for use in the present invention may consist of nucleotide sequences depicted in respective SEQ ID NOs or they may comprise additional nucleotides. Thus, they can be of different length, and only the desired specificity and functionality of these oligonucleotide probes determine their suitable length in hybridization reactions. The length of oligonucleotide probe sequence used in the method and the kit of the invention is preferably 15 to 60, more preferably 15 to 40, and most preferably 20 to 30 nucleotides. Furthermore, the probes can be modified in different ways (e.g., oligonucleotide analogues, such as peptide nucleic acids (PNA), or oligonucleotides comprising modified bases can be used) as long as they retain the species-specificity. Also, various chemical compounds or groups (e.g., amino groups) or other molecules, such as labels necessary for the detection, can be attached to the probes, or they can be entirely unmodified. Naturally, reverse and complementary sequences of these oligonucleotide sequences are equally suitable, as is obvious to a person skilled in the art.

Herein the term "complementary sequence" refers to a nucleic acid sequence which consists of complementary nucleotides to the original probe sequence. For example, the complementary sequence to C-A-T-G (where each letter stands for one of the bases in DNA) is G-T-A-C. Thus, the term "complementary sequence" refers to a completely complementary sequence which consists of as many nucleic acids as the original probe sequence.

The term "reverse sequence" refers to a nucleic acid sequence in which the order of nucleotides is reversed as compared to the original probe sequence. For example, reverse sequence to T-T-C-A-G (where each letter stands for one of the bases in DNA) is G-A-C-T-T. Thus, the term "reverse sequence" refers to a completely reversed nucleic acid sequence consisting of as many nucleic acids as the original non-reversed probe sequence.

Thus, each specific SEQ ID NO also refers to reversed and complementary sequences thereof. Similarly, functional fragments of the above mentioned oligonucleotide sequences are useful as probes. In this context, the term "functional fragment" refers to a truncated nucleotide sequence with or without modifications, capable of hybridizing with targeted bacteria and thus retaining unchanged species-specificity.

Disclosed is a mixture of oligonucleotide probes comprising any combination of the probes disclosed. In some embodiments, said mixture or combination may be gene-specific, species-specific, or genus-specific.

The present invention further provides a kit, as defined in claim 11, for use in the above method. Said kit comprises at least one topoisomerase-specific primer, at least one mecA-specific primer, at least one S. aureus-species-specific topoisomerase probe, and at least one mecA-specific oligonucleotide probe. Specific primers and probes for use in the kit are described above.

### Example 1. Detecting staphylococcus aureus and mecA gene

DNA extracted from a patient derived bacterial culture isolate of methicillin resistant *Staphylococcus aureus* (MRSA) was amplified using the method described below.

DNA was isolated from the sample to be analyzed using a nucleic acid extraction robot (Nuclisens® easyMAG™, bioMérieux, France). After DNA isolation, the desired target was amplified using PCR. First, a reaction solution was prepared. The PCR reaction mixture contained 1 µM of gB3F primer mixture (SEQ ID NO: 1; ordered from Thermo Electron, USA), 1 µM of Cy5-labeled gB4R primer mixture (SEQ ID NO: 2; ordered from Thermo Electron, USA), 0.25 µM *mecA* primer (SEQ ID NO: 5), 0.25 µM Cy5-labeled mecA primer (SEQ ID NO: 6), 0.165 µM of *S*. *aureus* specific primer (SEQ ID NO: 3), 1xHot Start Taq® PCR buffer (Qiagen, Germany), in which MgCl₂ was added so that the final concentration was 2.0 mM, 300 µM of each of dATP, dGTP, dCTP, and dTTP (Finnzymes, Finland), 1.5 g/l BSA (EuroClone, Italy), 0.125 U/µl Hot Start Taq® DNA polymerase (Qiagen, Germany), and 1.5 µl isolated DNA in a total volume of 15 µl.

The PCR was performed using a thermal cycler (Mastercycler®, Eppendorf, Germany). The following PCR program was used: a 15 min denaturation step at 95°C, 36 cycles of 20 sec at 95°C, 35 sec at 52°C, 20 sec at 72°C, after which 10 cycles of 10 sec at 95°C, 30 sec at 67°C, and finally 5 cycles of 10 sec at 95°C, 30 sec at 69°C. After the PCR, the success of amplification of DNA was verified by gel electrophoresis using a 2% agarose gel containing SYBR® Green II (Invitrogen, USA).

Amplified targets were hybridized on the silicon based biochip to which the probes were attached. The hybridization reaction mixture contained around 50 to 100 ng of labeled target, 2xhybridization buffer (consisting of 1 tablet PBS pH 7.4 (Sigma, USA), 0.7 M NaCl (Fluka, Switzerland), 0.1% Tween® 20 (100%, Fluka, Switzerland), 2xDenhardt's (5x, Sigma, USA), 20 µg/ml salmon sperm DNA (dsDNA) (Amersham, United Kingdom)), hybridization control oligonucleotides, and sterile water. The volume of the reaction mixture was 25 µl. The 2xhybridization buffer was pre-warmed to 55°C before use. The hybridization reaction mixture was transferred to a hybridization area of the biochip. The hybridization area was sealed with clamps and the biochip was placed in a special hybridization device. During 10 min hybridization at 55°C, a Cy5-labeled ssDNA target bound to the complementary probe sequence immobilized on the microarray creating a labeled double-stranded structure.

After the hybridization step, the biochip was briefly washed in order to remove non-hybridized DNA. The washing steps were carried out as follows: in 2xSSC solution for 2.5 min at 40°C, and in 0.2xSSC solution for 2.5 min at 40°C. After the washing, the biochip was dried. The biochip was analyzed with an optical reader, which consists of a LED light source and a CCD camera. The detection is based on a fluorescent signal released by the Cy5-labeled gene product and captured by the camera. An example of the hybridization result, illustrating the detection of an *S*. *aureus* and *mecA-gene,* is presented in Figure 1. No hybridization was observed with a *Staphylococcus* genus-specific probe, implying that the patient had an MRSA infection. The identification was in line with the result from blood culturing. Thus, the results demonstrate a rapid identification of methicillin-resistant *Staphylococcus aureus.*

### Example 2. Detecting staphylococcus and mecA gene

DNA, extracted from a pure bacterial culture of *Staphylococcus haemolyticus,* was amplified and hybridized on a microarray by methods described in Example 1.

The results revealed hybridization with *Staphylococcus* genus-specific probe as well as *mecA* gene probes. No hybridization was observed with an S. aureus-species-specific probe (Figure 2). The identification was in line with the original identification of the bacterial culture. Thus, based on the result it could be concluded that no specific treatment for MRSA was necessary.

## Claims

1. A method of detecting and identifying methicillin resistant *Staphylococcus* species in a sample, comprising the steps of:
a) amplifying DNA from said sample using at least one first primer pair which hybridizes with conserved regions of genes encoding topoisomerases, and a second primer pair which hybridizes with *mecA* gene; and
b) contacting the amplified DNA in hybridization conditions with
i) at least one *mecA*-specific oligonucleotide probe comprising a sequence selected from a group consisting of SEQ ID NOs: 7 to 12 and complementary sequences thereof,
ii) at least one *S*. *aureus-specific* oligonucleotide probe hybridizing with the amplified topoisomerase genes, said probe comprising a sequence selected from the group consisting of SEQ ID NOs: 13 to 20 and complementary sequences thereof, and
iii) at least one staphylococcus genus-specific oligonucleotide probe hybridizing with the amplified topoisomerase genes of staphylococcal species other than S.aureus; and
c) detecting any hybridization complexes formed and identifying said infection-causing bacteria based on said detection of said hybridization complex.

2. The method according to claim 1, further comprising contacting the amplified DNA with at least one *S*. *epidermidis* species-specific oligonucleotide probe hybridizing with the amplified topoisomerase genes, said probe comprising a sequence selected from a group consisting of SEQ ID NOs: 21 to 25 and complementary sequences thereof.

3. The method according to claim 1, wherein said first primer pair comprises at least one sequence depicted in SEQ ID NO: 1 or 2.

4. The method according to claim 1, wherein said second primer pair comprises at least one sequence selected from SEQ ID NO: 5 and 6.

5. The method according to claim 1, wherein said staphylococcus genus-specific probe comprises a sequence selected from a group consisting of SEQ ID NOs: 26 to 33 and complementary sequences thereof.

6. The method according to claim 1, wherein in step a) DNA amplification is further performed with at least one *S*. *aureus-specific* primer hybridizing with conserved regions of genes encoding topoisomerases, said primer comprising a sequence selected from SEQ ID NOs: 3 and 4.

7. The method according to claim 1, wherein said sample is an *S*. *aureus* pure culture, and said first primer pair is selected from SEQ ID NOs: 3 and 4.

8. The method according to any preceding claim, wherein said oligonucleotide probes are attached onto a solid support.

9. A *mecA*-specific oligonucleotide probe consisting of a nucleotide sequence selected from a group consisting of SEQ ID NOs: 7, 9-12 and complementary sequences thereof.

10. An *S*. *aureus-specific* oligonucleotide probe hybridizing with a hypervariable region of topoisomerase genes, said probe consisting of a sequence selected from the group consisting of SEQ ID NOs: 13-16, 19, 20 and complementary sequences thereof.

11. A kit for use in the method of claim 1, comprising
a) at least one topoisomerase-specific primer
b) at least one *mecA*-specific primer
c) at least one *S. aureus*-species-specific topoisomerase probe comprising a sequence selected from a group consisting of SEQ ID NOs: 13 to 20 and complementary sequences thereof,
d) at least one *mecA*-specific oligonucleotide probe consisting of a sequence selected from a group consisting of SEQ ID NOs: 7 to 12 and complementary sequences thereof, and
e) at least one staphylococcal genus-specific topoisomerase probe hybridising to staphylococcal species other than S.aureus and comprising a sequence selected from a group consisting of SEQ ID NOs: 26 to 33 and complementary sequences thereof.

12. The kit according to claim 11, wherein said topoisomerase-specific primer comprises at least one sequence depicted in SEQ ID NOs: 1 or 2 or in complementary sequences thereof.

13. The kit according to claim 11, further comprising at least one *S*. *epidermidis*-species-specific topoisomerase probe comprising a sequence selected from a group consisting of SEQ ID NOs: 21 to 25 and complementary sequences thereof.

14. The kit according to claim 11, wherein said topoisomerase-specific primer is *S. aureus*-species-specific primer comprising a sequence selected from SEQ ID NOs: 3 and 4 and complementary sequences thereof.

15. The kit according to any one of claims 11 to 14, further comprising at least one *S*. *aureus* species-specific topoisomerase-specific primer comprising a sequence selected from SEQ ID NOs: 3 and 4 and complementary sequences thereof.

16. The kit according to any one of claims 11 to 15, wherein said *mecA*-specific primer comprises a sequence selected from SEQ ID NOs: 5 and 6 and complementary sequences thereof.

17. The kit according to any one of claims 11 to 16, wherein said primers and probes are contained on a biochip.

## Patentansprüche

1. Ein Verfahren zum Detektieren und Identifizieren Methicillin-resistenter Staphylokokkus-Spezies in einer Probe, umfassend die Schritte:
a) Amplifizieren von DNA aus dieser Probe unter Verwendung mindestens eines ersten Primer-Paares, das mit den konservativen Genregionen, die Topoisermerasen kodieren, hybridisiert und eines zweiten Primer-Paares, das mit dem *mecA* Gen hybridisiert, und
b) Inkontaktbringen der amplifizierten DNA unter Hybridisierungsbedingungen mit
i. mindestens einer *mecA*-spezifischen Oligonukleotid-Sonde, die eine Sequenz ausgewählt aus der Gruppe bestehend aus SEQ ID Nr. 7 bis 12 und komplementären Sequenzen davon umfasst,
ii. mindestens einer *S. aureus*-spezifischen Oligonukleotid-Sonde, die mit den amplifizierten Topoisomerasegenen hybridisiert, wobei die Probe eine Sequenz ausgewählt aus der Gruppe bestehend aus SEQ ID Nr. 13 bis 20 und komplementären Sequenzen davon umfasst und
iii. mindestens einer Staphylokokkus-Genus-spezifischen Oligonukleotid-Sonde, die mit den amplifizierten Topoisomerasegenen anderer Staphylokokken-Spezies als *S. aureus* hybridisiert, und
c) Detektieren gebildeter Hybridisierungskomplexe und Identifizieren der infektionsauslösenden Bakterien, basierend auf dieser Detektion des Hybridisierungskomplexes.

2. Das Verfahren nach Anspruch 1, weiterhin umfassend das Inkontaktbringen der amplifizierten DNA mit mindestens einer *S. epidermis*-Spezies-spezifischen Oligonukleotid-Sonde, die mit den amplifizierten Topoisomerasegenen hybridisiert, wobei die Probe eine Sequenz ausgewählt aus der Gruppe bestehend aus SEQ ID Nr. 21 bis 25 und komplementären Sequenzen davon umfasst.

3. Das Verfahren nach Anspruch 1, wobei das erste Primer-Paar mindestens eine Sequenz umfasst, die in SEQ ID Nr. 1 oder 2 dargestellt ist.

4. Das Verfahren nach Anspruch 1, wobei das zweite Primer-Paar mindestens eine Sequenz ausgewählt aus SEQ ID Nr. 5 und 6 umfasst.

5. Das Verfahren nach Anspruch 1, wobei die Staphylokokken-Genus-spezifische Sonde eine Sequenz ausgewählt aus der Gruppe bestehend aus SEQ ID Nr. 26 bis 33 und komplementären Sequenzen davon umfasst.

6. Das Verfahren nach Anspruch 1, worin in Schritt a) ferner eine DNA-Amplifizierung mit mindestens einem *S. aureus*-spezifischen Primer, der mit konservativen Genregionen, die Topoisomerasen kodieren, hybridisiert, durchgeführt wird, wobei der Primer eine Sequenz ausgewählt aus SEQ ID Nr. 3 und 4 umfasst.

7. Das Verfahren nach Anspruch 1, wobei die Probe eine *S. aureus*-Reinkultur ist, und das erste Primer-Paar ausgewählt ist aus SEQ ID Nr. 3 und 4.

8. Das Verfahren nach einem der voranstehenden Ansprüche, wobei die Oligonukleotid-Sonden an einen festen Träger gebunden sind.

9. Eine *mecA*-spezifische Oligonukleotid-Sonde bestehend aus einer Nukleotidsequenz, die aus der Gruppe bestehend aus der SEQ ID Nr. 7, 9 bis 12 und komplementären Sequenzen davon ausgewählt ist.

10. Eine *S. aureus*-spezifische Oligonukleotid-Sonde, die mit einer hypervariablen Region von Topoisomerasegenen hybridisiert, wobei die Sonde aus einer Sequenz ausgewählt aus der Gruppe bestehend aus SEQ ID Nr. 13 bis 16, 19, 20 und komplementären Sequenzen davon besteht.

11. Ein Kit zur Verwendung in dem Verfahren nach Anspruch 1, umfassend
a) mindestens einen Topoisomerase-spezifischen Primer,
b) mindestens einen *mecA*-spezifischen Primer,
c) mindestens eine *S. aureus*-Spezies-spezifische Topoisomerase-Sonde, die eine Sequenz ausgewählt aus der Gruppe bestehend aus SEQ ID Nr. 13 bis 20 und komplementären Sequenzen davon umfasst,
d) mindestens eine *mecA*-spezifische Oligonukleotid-Sonde bestehend aus einer Sequenz ausgewählt aus der Gruppe bestehend aus den SEQ ID Nr. 7 bis 12 und komplementären Sequenzen davon, und
e) mindestens eine *S. aureus*-Spezies-spezifische Topoisomerase-Sonde, die mit anderen Staphylokokken-Spezies als *S. aureus* hybridisiert, und die eine Sequenz ausgewählt aus der Gruppe bestehend aus den SEQ ID Nr. 26 bis 33 und komplementären Sequenzen davon umfasst.

12. Das Kit nach Anspruch 11, wobei der Topoisomerase-spezifische Primer mindestens eine Sequenz umfasst, die in SEQ ID Nr. 1 oder 2 oder komplementären Sequenzen davon dargestellt ist.

13. Das Kit nach Anspruch 11, weiterhin umfassend mindestens eine *S. epidermis-*Spezies-spezifische Topoisomerase-Sonde, die eine Sequenz ausgewählt aus der Gruppe bestehend aus SEQ ID Nr. 21 bis 25 und komplementären Sequenzen davon umfasst.

14. Das Kit nach Anspruch 11, wobei der Topoisomerase-spezifische Primer *S. aureus*-Spezies-spezifischer Primer ist, der eine Sequenz ausgewählt aus den SEQ ID Nr. 3 und 4 und komplementären Sequenzen davon umfasst.

15. Das Kit nach einem der Ansprüche 11 bis 14, weiterhin umfassend mindestens einen *S. aureus*-Spezies-spezifischen Topoisomerase-spezifischen Primer, welcher eine Sequenz ausgewählt aus den SEQ ID Nr. 3 und 4 und komplementären Sequenzen davon umfasst.

16. Das Kit nach einem der Ansprüche 11 bis 15, wobei der *mecA*-spezifische Primer eine Sequenz, ausgewählt aus den SEQ ID Nr. 5 und 6 und komplementären Sequenzen davon, umfasst.

17. Das Kit nach einem der Ansprüche 11 bis 16, wobei die Primer und Sondsen auf einem Biochip enthalten sind.

## Revendications

1. Procédé de détection et d'identification d'une espèce de *Staphylococcus* résistant à la méticilline dans un échantillon, comprenant les étapes qui consistent à :
a) amplifier de l'ADN à partir dudit échantillon en utilisant au moins une première paire d'amorces qui s'hybride avec des régions conservées de gènes codant pour des topoisomérases, et une seconde paire d'amorces qui s'hybride avec le gène *mecA* ; et
b) mettre en contact l'ADN amplifié dans des conditions d'hybridation avec
i) au moins une sonde oligonucléotidique spécifique à *mecA* comprenant une séquence sélectionnée dans un groupe consistant en SEQ ID NO: 7 à 12 et des séquences complémentaires de celles-ci,
ii) au moins une sonde oligonucléotidique spécifique à *S*. *aureus* qui s'hybride avec les gènes de topoisomérase amplifiés, ladite sonde comprenant une séquence sélectionnée dans le groupe consistant en SEQ ID NO: 13 à 20 et des séquences complémentaires de celles-ci, et
iii) au moins une sonde oligonucléotidique spécifique au genre staphylococcus qui s'hybride avec les gènes de topoisomérase amplifiés d'une espèce de staphylocoque autre que S. aureus ; et
c) détecter de quelconques complexes d'hybridation formés et identifier lesdites bactéries provoquant une infection en se basant sur ladite détection dudit complexe d'hybridation.

2. Procédé selon la revendication 1, comprenant en outre la mise en contact de l'ADN amplifié avec au moins une sonde oligonucléotidique spécifique à l'espèce *S*. *epidermidis* qui s'hybride avec les gènes de topoisomérase amplifiés, ladite sonde comprenant une séquence sélectionnée dans un groupe consistant en SEQ ID NO: 21 à 25 et des séquences complémentaires de celles-ci.

3. Procédé selon la revendication 1, dans lequel ladite première paire d'amorces comprend au moins une séquence représentée dans SEQ ID NO: 1 ou 2.

4. Procédé selon la revendication 1, dans lequel ladite seconde paire d'amorces comprend au moins une séquence sélectionnée parmi SEQ ID NO: 5 et 6.

5. Procédé selon la revendication 1, dans lequel ladite sonde spécifique au genre staphylococcus comprend une séquence sélectionnée dans un groupe consistant en SEQ ID NO: 26 à 33 et des séquences complémentaires de celles-ci.

6. Procédé selon la revendication 1, dans lequel à l'étape a), l'amplification de l'ADN est en outre réalisée avec au moins une amorce spécifique à *S*. *aureus* qui s'hybride avec des régions conservées de gènes codant pour des topoisomérases, ladite amorce comprenant une séquence sélectionnée parmi SEQ ID NO: 3 et 4.

7. Procédé selon la revendication 1, dans lequel ledit échantillon est une culture pure de *S*. *aureus,* et ladite première paire d'amorces est sélectionnée parmi SEQ ID NO: 3 et 4.

8. Procédé selon l'une quelconque des revendications précédentes, dans lequel lesdites sondes oligonucléotidiques sont attachées sur un support solide.

9. Sonde oligonucléotidique spécifique à *mecA* consistant en une séquence de nucléotides sélectionnée dans un groupe consistant en SEQ ID NO: 7, 9-12 et des séquences complémentaires de celles-ci.

10. Sonde oligonucléotidique spécifiques à *S*. *aureus* qui s'hybride avec une région hypervariable de gènes de topoisomérase, ladite sonde consistant en une séquence sélectionnée dans le groupe consistant en SEQ ID NO: 13-16, 19, 20 et des séquences complémentaires de celles-ci.

11. Kit destiné à être utilisé dans le procédé selon la revendication 1, comprenant
a) au moins une amorce spécifique à une topoisomérase
b) au moins une amorce spécifique à *mecA*
c) au moins une sonde de topoisomérase spécifique à l'espèce *S*. *aureus* comprenant une séquence sélectionnée dans un groupe consistant en SEQ ID NO: 13 à 20 et des séquences complémentaires de celles-ci,
d) au moins une sonde oligonucléotidique spécifique à *mecA* consistant en une séquence sélectionnée dans un groupe consistant en SEQ ID NO: 7 à 12 et des séquences complémentaires de celles-ci, et
e) au moins une sonde de topoisomérase spécifique au genre staphylocoque qui s'hybride avec une espèce de staphylocoque autre que S. aureus et comprenant une séquence sélectionnée dans un groupe consistant en SEQ ID NO: 26 à 33 et des séquences complémentaires de celles-ci.

12. Kit selon la revendication 11, dans lequel ladite amorce spécifique à une topoisomérase comprend au moins une séquence représentée dans SEQ ID NO: 1 ou 2 ou dans des séquences complémentaires de celles-ci.

13. Kit selon la revendication 11, comprenant en outre au moins une sonde de topoisomérase spécifique à l'espèce *S*. *epidermidis* comprenant une séquence sélectionnée dans un groupe consistant en SEQ ID NO: 21 à 25 et des séquences complémentaires de celles-ci.

14. Kit selon la revendication 11, dans lequel ladite amorce spécifique à une topoisomérase est une amorce spécifique à l'espèce *S*. *aureus* comprenant une séquence sélectionnée parmi SEQ ID NO: 3 et 4 et des séquences complémentaires de celles-ci.

15. Kit selon l'une quelconque des revendications 11 à 14, comprenant en outre au moins une amorce spécifique à une topoisomérase spécifique à l'espèce *S*. *aureus* comprenant une séquence sélectionnée parmi SEQ ID NO: 3 et 4 et des séquences complémentaires de celles-ci.

16. Kit selon l'une quelconque des revendications 11 à 15, dans lequel ladite amorce spécifique à *mecA* comprend une séquence sélectionnée parmi SEQ ID NO: 5 et 6 et des séquences complémentaires de celles-ci.

17. Kit selon l'une quelconque des revendications 11 à 16, dans lequel lesdites amorces et sondes sont contenues sur une biopuce.
